Europäisches Patentamt

European Patent Office

Office européen des brevets

(11) Publication number: **0 312 222 B1**

## EUROPEAN PATENT SPECIFICATION

(45) Date of publication of patent specification: **05.08.92**

(51) Int. Cl.5: **A61K 31/725**, A61K 31/70, //(A61K31/725,31:70)

(21) Application number: **88308972.4**

(22) Date of filing: **28.09.88**

(54) Treatment of diseases caused by viruses.

(30) Priority: **14.10.87 JP 259919/87**

(43) Date of publication of application:
**19.04.89 Bulletin 89/16**

(45) Publication of the grant of the patent:
**05.08.92 Bulletin 92/32**

(84) Designated Contracting States:
**AT BE CH DE ES FR GB GR IT LI LU NL SE**

(56) References cited:
**EP-A- 0 270 317**
**US-A- 4 678 772**

**CHEMICAL ABSTRACTS, vol. 107, no. 23, 7th December 1987, page 24, no. 211581f, Columbus, Ohio, US; H. NAKASHIMA et al.: "A new anti-human immuno-deficiency virus substance, glycyrrhizin sulfate: endowment of glycyrrhizin with reverse transcriptase-inhibitory activity by chemical modification", & JPN. J. CANCER RES. (GANN) 1987, 78(8), 767-71**

**Yamamoto et al. ARCH OF AIDS RESEARCH; (1), 1987, Ueno et al. LANCET, 1, 1987,**

**Loza et al. CHEM. ABSTR. 72(21), 1970, Abst. no. 109118g**

(73) Proprietor: **Kabushiki Kaisha Ueno Seiyaku Oyo Kenkyujo**
**2-31, Koraibashi**
**Higashi-ku Osaka-shi Osaka-fu(JP)**

(72) Inventor: **Ueno, Ryuzo**
**10-27, Nango-cho**
**Nishinomiya-shi Hyogo-ken(JP)**
Inventor: **Ueno, Ryuji**
**7-29, Misaku-cho**
**Nishinomiya-shi Hyogo-ken(JP)**
Inventor: **Kuno, Sachiko**
**11-4-603, 1-chome, Sakasedai**
**Takarazuka-shi Hyogo-ken(JP)**

(74) Representative: **Atkinson, Peter Birch et al**
**Marks & Clerk Suite 301 Sunlight House**
**Ouay Street**
**Manchester M3 3JY(GB)**

EP 0 312 222 B1

## Description

The present invention relates to a pharmaceutical composition (including pharmaceutical composition for veterinary use) containing as an active ingredient a combination of an antiviral agent of the nucleic acid type having no saccharide residue acylated with S-oxoacid with a certain glycoside having at least one S-oxoacid group, which is useful in the treatment of diseases caused by viruses. The combination as described above produces antiviral synergism and consequently permit enhancement of antiviral effect, while reducing individual dosages and alleviating adverse side-effects.

Very few chemotherapeutic agent for viral diseases have been available which can exert satisfactory therapeutic effects, in contrast to those for bacterial diseases. The compounds, which are effective against viruses in vitro, often produce severe adverse side-effects when administered to a living body, and even antiviral agents that are considered as tolerable for practical use find, in many instances, severely restricted application because of their adverse side-effects. This may be reflected, for example, by such antiviral agents as arabinosyladenine (Ara A) or Acyclovir being in recent years found to be effective against herpesviruses and azidothymidine (AZT) being recently reported to possess effects against AIDS (acquired immunodeficiency syndrome) related viruses (that is to say, human Immunodeficiency virus ordinarily referred to briefly as "HIV"); these antiviral agents when administered to humans cause side-effects such as nausea, emesis, diarrhoea, eruption, anemia and phlebitis developed locally at the site of administration, which sometimes leads to forced discontinuation of administration. Under these circumstances, development of antiviral agents with reduced side effects is strongly demanded and reduction of side-effects inherent to the conventional antiviral agents currently constitutes one of the most important research subjects since this offers better chance of success.

During the course of an investigation on synergism between various anti-viral agents, the present inventors have attempted a combined use of sulphated glycosides such as glycyrrhizin sulfate which was recently reported as having certain antiviral effect, as well as digitonin sulfate or stevioside sulfate, with the conventional antiviral agents. This combination has shown a significant synergistic activity of 20 to 50 times greater than that could be expected from simple addition of the individual drug effects. This finding has led us to a valuable pharmaceutical composition based on a new synergistic combination and use thereof for the preparation of a medicament which is useful in the treatment of virus disease, which is the subject of the present invention.

## 2. Description of the related art

A comprehensive review on antiviral agents is given in Kirk Othmer's Encyclopedia of Chemical Technology, vol. 5, 542-552. H. Nakashima, et al. (Jpn. J. Cancer Res. (Gann) 78, 767-771, Aug., 1987) described the antiviral activity of glycyrrhizin sulfate against HIV.

Arch of Aids Research, Vol. 1, No. 1, 1987, pages 45-56 discloses that several sulfated polysaccharides and sulfated glycyrrhizin show, in contrast to their non-sulfated parent compounds, a strong anti-HIV activity. The Lancet, Vol. 1, 19 June 1987, page 1379 discloses that the sulfate residues may play a key role in the anti-HIV activity of these compounds. It has also been disclosed that sulfated polysaccharides potentiate the antiviral activity of other antiviral agents, such as interferon (see Chemical Abstracts, Vol. 72, No. 21, 15 May 1970, page 195, Abstract No. 109118g), ATZ (see the aforementioned article in The Lancet) and other nucleic acid type antiviral agents (see EP-A-0 270 317).

## SUMMARY OF THE INVENTION

The present invention provides the use of a composition comprising (a) a nucleic acid type antiviral agent and (b) a glycoside acylated with S-oxoacid, said glycoside being selected from phenol glycosides, nitrile glycosides, coumarin glycosides, anthracene glycosides, terpene glycosides, bitterness glycosides, flavone glycosides, isoflavone glycosides, flavonol glycosides, flavanoe glycosides, pelargonidin glycosides, cyanidin glycosides, delphinidin glycosides, steroid glycosides, triterpenoid glycosides, cardiotonic glycosides, indoxyl glycosides, gibberellin glycosides, S-glycosides, C-glycosides and C,O-mixed glycosides, or a pharmaceutically acceptable salt of such glycosides for the manufacture of a medicament for the treatment of a disease caused by a retrovirus.

## DESCRIPTION OF THE PREFERRED EMBODIMENT

The term "treatment" as used herein is intended to include all the handling of diseases such as

EP 0 312 222 B1

prevention, sustention (i.e. prevention of aggravation), alleviation(i.e. amelioration of conditions) and therapy.

The viral diseases to be treated according to the present invention include diseases caused by various viruses. Such viruses include Retroviridae (e.g. HIVs (e.g. HTLV-III, LAV, ARV etc.), ovine visna virus, equine infectious anemia virus, avian leukemia virus, avian sarcoma virus, avian reticuloendothetiosis virus, murine breast carcinoma virus, murine leukemia virus, murine sarcoma virus, equine type C virus, hamster type C virus, rat leukemia virus, feline leukemia virus, feline sarcoma virus, feline type C virus, ovine leukemia virus, bovine leukemia virus, swine type C virus, simian leukemia virus, Mason-Pfizer Virus, simian sarcoma virus, simian T-lymphotropic virus, baboon type C virus, adult T-cell leukemia virus (ATLV) or human T-lymphotropic virus types I and II (HTLV-I, HTLV-II) or human Kawasaki disease virus. The important diseases are AIDS, PGL (Presistent generalized lymphadenopathy), ARC(AIDS-related complex), LAS(Lymphadenopathy syndrome) and human T-cell leukemia.

The nucleic acid type antiviral agent (a) (which is an anti-viral agent having no saccharide residue acylated with S-oxoacid) which is useful in this invention includes for example, 3'-azido-3'-deoxythymidine (azidothymidine or AZT), 2',3'-dideoxynucleosides (2',3'-dideoxyadenosine, -guanosine, -inosine, -cytidine, or -thymidine), ribavirin, isoprinosin, acyclovir, Ara A, Ara T, Ara C, iododeoxyuridine (IDU), bromovinyl deoxyuridine, fluoroiodo aracytosin, 2'-amino-2'-deoxyribofuranosyladenine, trifluridine, acyclovir derivatives (deoxy-, glycyl- or iodo-acyclovir), dihydroxypropoxymethyl guanine, dihydroxybutyl guanine), chloroethyldeoxyuridine, toyocamycin or puromycin.

The term "saccharide esterified with S-oxoacid" has the meaning of a saccharide residue having at least one S-oxoacid group in "glycoside acylated with S-oxoacid" as described below.

Preferred nucleic acid type antiviral agents are those having reverse transcriptase inhibitory activity and effective against HIV (AZT, 2',3'-dideoxynucleosides, and other agents not having reverse transcriptase inhibitory activity but effective against HIVs (ribavirin).

The term "glycoside acylated with S-oxoacid" as used herein refers to glycosides having not less than two monosaccharide residues where not less than one of the monosaccharide residues has(have) at least one S-oxoacid group attached to the saccharic carbon atom(s) thereof through a linking group with a low molecular weight. The glycosides acylated with S-oxoacid used in the present invention include natural or synthetic glycosides which have not less than two (e.g. 2, 3, 4, 5 or more) saccharide residues having S-oxoacid group attached to the saccharic carbon atom thereof through a linking group with a low molecular weight.

The term "glycoside" refers to saccharide derivatives in which a non-saccharide group (aglycone) is attached by the atom, such as oxygen, nitrogen or sulphate.

The term "natural glycoside" means glycosides which can be obtained by extraction from natural resources, such as plants, microbes or animals.

The term "synthetic glycoside" refers to glycosides which can be obtained by binding one or more saccharic group onto an aglycone, a glycoside having one monosaccharic residue or a glycoside having not less than two monosacharic residues by chemical synthesis or biochemical synthesis (e.g. by an enzyme).

The natural or synthetic glycosides used in the present invention are selected from phenol glycosides, nitrile glycosides, coumarin glycosides, anthracene glycosides, terpene glycosides, bitterness glycosides, flavone glycosides, isoflavone glycosides, flavonol glycosides, flavanoe glycosides, pelargonidin glycosides, cyanidin glycosides, delphinidin glycosides, steroid glycosides, triterpenoid glycosides, cardiotonic glycosides, indoxyl glycosides, gibberellin glycosides, S-glycosides, C-glycosides or C,O-mixed glycosides, and important among them are triterpenoid glycosides, steroid glycosides and terpene glycosides.

Examples of glycosides include lotusin , sennoside A or B, stevioside, apiin, kaempferitrin, kaempferin, butrin, salvianin, pelargonin, monardein, cyanin, peonin, mecocyanin, lycoricyanin, delphinin, delphin, nasunin, hyacin, violanin, malvin, amolonin, osladin, kammonin, sarsasaponin, dioscin, digitonin, gitonin, convallasaponin C or D, glucoconvallasaponin A or B, tigonin, asiaticoside, aescin, glycyrrhizin, cyclamin, theasaponins, camellia saponins, $\alpha$-hederin, spirasaponin A or B, solasonine, solanine, tomatine, lucenin-1, xylosylglucosyl apigenin and saponarin.

The glycoside acylated with S-oxoacid may have at least one but normally not more than four S-oxoacid groups on at least one, a minor portion, a major portion or all of the constituent monosaccharides.

The S-oxoacid group includes sulfo group ($-SO_3H$) and hydroxysulfinyl group ($-SO.OH$), with sulfo group being preferred.

The term "acylated" includes "esterified", "amidated" and "C-acylated".

The term "saccharic carbon atom" refers to a carbon atom which constitutes a chain skeleton, a tetrahydrofuran ring or tetrahydropyran ring contained in saccharides.

The term "low-molecular-weight linking group" is intended to comprehend oxy ($-O-$), imino ($-NH-$), thio ($-S-$), methylene ($-CH_2-$) or ethylidene ($-CH(CH_3)-$) groups. The term "lower molecular weight" means

3

molecular weights of any linking groups ranging from 14 to 32. The preferred linking group includes oxy and imino.

The saccharides (serving as a repeating unit) include, xylose, arabinose, rhamnose, fucose, glucose, galactose, glucuronic acid, galacturonic acid or mannuronic acid.

Among such glycosides acylated with S-oxoacid are included known ones and novel ones. Such novel glycosides acylated with S-oxoacid can be produced by the same procedure as empolyed for known ones, with an example of such production procedures being described in the following.

Chlorosulfonic acid is added dropwise to dry pyridine in volume 8 to 10 times that of chlorosulfonic acid, while cooling, and a small amount each of formamide and a glycoside (in amounts about one-fourth of chlorosulfonic acid) are added to the mixture, followed by heating at 55 - 65° C under stirring. After stirring the mixture for several hours, the solvent is distilled off, and the residue is purified for example by reprecipitation or recrystallization.

The term "pharmaceutically acceptable salts" is intended to designate salts which retain biological activity of the parent compounds but do not exhibit any adverse toxicity at normal dose levels. Such salts include the salts of inorganic bases, such as sodium, potassium, and ammonium salts, salts with organic bases, such as diethanolamine and amino acid salts. These salts can be produced from their corresponding free acids.

The nucleic acid type antiviral agent and the glycoside acylated with S-oxoacid may be administered concurrently or sequentially. However, both of them should in principle be administered on the same day, optionally at different times in the same day but as close to each other as possible. Naturally, these two kinds of active ingredients may be administered simultaneously in the form of a combination of their individual pharmaceutical preparations or they may be contained in the same, as a single pharmaceutical preparation. Such combination is optionally selected depending upon the conditions of patients or kind of diseases.

The dosage of the nucleic acid type antiviral agent may be such an amount as to be sufficient to produce a concentration permitting development of antiviral activity in body, even when it is administered alone. Nevertheless, since the present invention produces synergism, the antiviral agent may in some instances be given in doses less than the amount required to produce the above described concentration, for example, 1/2 to 1/200, 1/10 to 1/100 or 1/20 to 1/50 of the said amount. The specific dosage can vary largely depending upon the type and nature of the individual pharmaceutical preparations, and may be changed with the conditions of patients, kind of diseases, and type and amount of the glycoside acylated with S-oxoacid to be combined.

The glycosides acylated with S-oxoacid include compounds having or lacking antiviral activity. The dosage of the acylated glycoside(s) having antiviral activity may be such an amount as to be sufficient to produce a concentration permitting development of antiviral activity in body, even when it is administered solely. However, since the present invention produces antiviral synergism, the acylated glycoside(s) may be in some instances be given in doses less than the amount producing the above-described concentration, for example, 1/2 to 1/500, 1/5 to 1/200, or 1/20 to 1/50 of the said amount. The specific dosage of the acylated glycoside(s) may in any case vary depending on the nature of individual acylated glycoside(s), condition of patients, kind of diseases and type and amount of the antiviral agent to be combined, and generally ranges from 0.02 to 200 mg/kg, preferably 0.1 - 100 mg/kg, with the acylated glycoside(s) being administered to human being at a daily dose of about 1 mg to 10 g/day, preferably about 5 mg to 5 g/day.

Each of the above pharmaceutical preparations may be administered once a day or as divided into two to six times or as a sustained release dosage form.

The administration can be made through any optional routes, such as by oral and topical application and injection.

For administration, the active ingredients may be mixed with a pharmaceutical carrier, such as organic or inorganic solid or liquid excipient, being suitable for such administration route as oral administration or injection, processed in the form of the conventional pharmaceutical preparations. Such pharmaceutical preparations include solid ones, such as tablets, granules, powders and capsules, and liquid ones such as solutions, emulsions and suspensions. The above carriers include, for example, starch, lactose, glucose, sucrose, dextrins, celluloses, paraffins, fatty acid glycerides, water, alcohols or gum arabic. If necessary, there may be added adjuvants, stabilizers, emulsifiers, lubricants, binders, pH-regulating agents, isotonic agents, and other conventional additives.

The following examples will illustrate the present invention in further detail.

The examples are given below to illustrate this invention in more detail, with the test examples being also described to clarify the effects of this invention.

Preparation 1

Preparation of sulfated glycyrrhizin

Glycyrrhizin (5g) was added to 95 % sulfuric acid (10 ml) cooled to below -25°C with stirring. After the reaction mixture was stirred at the same temperature for 90 minutes, the reaction solution was gradually poured onto ice (120 g) with stirring. To the resulting solution was gradually added calcium carbonate with well stirring. The precipitates were filtered off with suction and then washed. Ethanol was added to the combined filtrates to the final concentration of 20%(v/v), and the resulting solution was kept to stand overnight at 5 °C to precipitate calcium sulphate. The precipitates were filtered off, and the filtrate was adjusted to pH 10 with sodium carbonate. After addition of acetic acid to make the solution weakly acidic, the solution was concentrated to about 20 ml, then diluted with ethanol (100 ml), and kept to stand overnight at 5 °C. The precipitates formed in the solution were isolated with centrifugation, washed with ethanol, and with ether, and dried under vaccum to give the white powders of the title compound.

Example 1

| (A) Sodium glycyrrhizin sulfate | 300 mg |
| Corn starch | 45 " |
| Lactose | 300 " |
| Magnesium stearate | 5 " |

The above ingredients are mixed, granulated and pressed into tablets and according to the conventional procedure.

| (B) 3'-Azido-3'-deoxythymidine | 25 mg |
| Corn starch | 120 " |
| Lactose | 300 " |
| Magnesium stearate | 5 " |

The above ingredients are mixed and filled in hard gelatine capsules and according to the conventional procedure.
(A) and (B) are combined into a dose form.

Example 2

| Sodium glycyrrhizin sulfate | 120 mg |
| 3'-Azido-3'-deoxythymidine | 100 " |
| 0.9 % saline | q.s. to 10 ml |

The above ingredients are mixed and dissolved according to the conventional procedure to form an injectable solution.

Example 3

| Sodium stevioside sulfate | 200 units |
|---|---|
| 3'-Azido-3'-deoxythymidine | 50 mg |
| 0.9 % Saline | q.s. to 10 ml |

Example 4

| Sodium digitonin sulfate | 500 units |
|---|---|
| Ara C | 10 mg |
| Procain hydrochloride | 10 mg |
| Water | q.s. to 10 ml |

Example 5 (Inhibition of viral infection)

AIDS virus HTLV-III/LAV was challenged to established human T-lymphocyte culture cells MT-4 and the cell suspension and virus particles were incubated at 37°C for 1 hour. The cells were then washed with phosphate buffered saline once and cultured with or without various doses of the test substances in RPMI-1640 medium at 37°C under 5 % $CO_2$ in an incubator.

MT-4 cells infected with HTLV-III were destructed as the virus proliferated, resulting in decrease of viable cells. Prior to the cytolysis by viral cytopathic effect, the cells infected with HTLV-III appeared which were detectable by indirect immuno fluorescence method. On day 12, the number of the living cells was counted to determine the extent of infection, and the rate of the infected cells was determined by indirect immuno-fluorescence method using an antibody having specificity to the virus specific antigen. As the test substances, 3'-azido-3'-deoxythymidine (AZT), glycyrrhizin sulfate, digitonin sulphate and stevioside sulfate were used. The results are shown in the following table.

| AZT glycyrrhizin sulfate($\mu$g/ml) | 0nM | 10nM | 50nM |
|---|---|---|---|
| 0 | 100 | 100 | 100 |
| 100 | 100 | 80 | 80 |
| 250 | 100 | 60 | 25 |

| AZT digitonin sulfate($\mu$g/ml) | 0nM | 10nM | 50nM |
|---|---|---|---|
| 0 | 100 | 100 | 100 |
| 100 | 100 | 60 | 60 |
| 250 | 80 | 60 | 10 |

| AZT stevioside sulfate($\mu$g/ml) | 0nM | 10nM | 50nM |
|---|---|---|---|
| 0 | 100 | 100 | 100 |
| 100 | 100 | 80 | 40 |
| 250 | 80 | 50 | 10 |

It is evident from these results that the combined use of the above two substances produces a significant synergistic effect.

**Claims**

6

**Claims for the following Contracting States : AT, BE, CH, DE, FR, GB, IT, LI, LU, NL, SE**

1. The use of a composition comprising (a) a nucleic acid type antiviral agent and (b) a glycoside acylated with S-oxoacid, said glycoside being selected from phenol glycosides, nitrile glycosides, coumarin glycosides, anthracene glycosides, terpene glycosides, bitterness glycosides, flavone glycosides, isoflavone glycosides, flavonol glycosides, flavanoe glycosides, pelargonidin glycosides, cyanidin glycosides, delphinidin glycosides, steroid glycosides, triterpenoid glycosides, cardiotonic glycosides, indoxyl glycosides, gibberellin glycosides, S-glycosides, C-glycosides and C,O-mixed glycosides, or a pharmaceutically acceptable salt of such glycosides for the manufacture of a medicament for the treatment of a disease caused by a retrovirus.

2. The use according to claim 1, wherein the said antiviral agent is present in an amount lower than its antivirally effective dose by itself.

3. The use according to claim 1, wherein the said nucleic acid type antiviral agent is selected from 3'-azido-3'-deoxythymidine (AZT), 2'3'-dideoxythymidine, 2',3'-dideoxyadenosine, 2',3'-dideoxyguanosine, 2',3'-dideoxyinosine, 2',3'-dideoxycytidine, Ara A, Ara C, Ara T, iododeoxyuridine and Acyclovir.

4. The use according to claim 1, wherein not less than two saccharide residues in the glycoside are present in succession.

5. The use according to claim 1, wherein the glycoside is selected from (i) triterpenoid glycoside, (ii) steroid glycoside and (iii) terpene glycoside.

6. The use according to claim 5, wherein the said glycoside is selected from (i) glycyrrhizin, (ii) digitonin and (iii) stevioside.

7. The use according to claim 1, wherein the said S-oxoacid group is a sulfo group ($-SO_3H$).

8. The use according to claim 1, wherein the glycoside contains an oxy group (-0-) as the linking group between the aglycone and the saccharide residue.

9. The use according to claim 1, wherein the retrovirus is human retrovirus.

10. The use according to claim 9, wherein the human retrovirus is selected from HTLV-I, HTLV-II, HTLV-III, LAV, ARV and Kawasaki disease virus.

11. The use according to claim 1, wherein the viral disease is Lymphadenopathy syndrome (LAS), Persistent generalized lymphadenopathy (PGL), AIDS, AIDS-related complex (ARC), adult T-cell leukemia (ATL) or Kawasaki disease.

12. The use according to claim 1, wherein the said nucleic acid type antiviral agent (a) and said glycoside (b) are administered simultaneously and/or sequentially.

**Claims for the following Contracting States : ES, GR**

1. A method of preparing a composition for the treatment of a disease caused by a retrovirus which comprises mixing (a) a nucleic acid type antiviral agent and (b) a glycoside acylated with S-oxoacid, said glycoside being selected from phenol glycosides, nitrile glycosides, coumarin glycosides, anthracene glycosides, terpene glycosides, bitterness glycosides, flavone glycosides, isoflavone glycosides, flavonol glycosides, flavanoe glycosides, pelargonidin glycosides, cyanidin glycosides, delphinidin glycosides, steroid glycosides, triterpenoid glycosides, cardiotonic glycosides, indoxyl glycosides, gibberellin glycosides, S-glycosides, C-glycosides and C,O-mixed glycosides, or a pharmaceutically acceptable salt of such glycosides.

2. The method according to claim 1, wherein the said antiviral agent is present in an amount lower than its antivirally effective dose by itself.

3. The method according to claim 1, wherein the said nucleic acid type antiviral agent is selected from 3'-azido-3'-deoxythymidine (AZT), 2',3'-dideoxythymidine, 2',3'-dideoxyadenosine, 2',3'-dideoxyguanosine, 2',3'-dideoxyinosine, 2',3'-dideoxycytidine, Ara A, Ara C, Ara T, iododeoxyuridine and Acyclovir.

4. The method according to claim 1, wherein not less than two saccharide residues in the glycoside are present in succession.

5. The method according to claim 1, wherein the glycoside is selected from (i) triterpenoid glycoside, (ii) steroid glycoside and (iii) terpene glycoside.

6. The method according to claim 5, wherein the said glycoside is selected from (i) glycyrrhizin, (ii) digitonin and (iii) stevioside.

7. The method according to claim 1, wherein the said S-oxoacid group is a sulfo group (-SO$_3$H).

8. The method according to claim 1, wherein the glycoside contains is an oxy group (-0-) as the linking group between the aglycone and the saccharide residue.

9. The method according to claim 1, wherein the retrovirus is human retrovirus.

10. The method according to claim 9, wherein the human retrovirus is selected from HTLV-I, HTLV-II, HTLV-III, LAV, ARV and Kawasaki disease virus.

11. The method according to claim 1, wherein the viral disease is Lymphadenopathy syndrome (LAS), Persistent generalized lymphadenopathy (PGL), AIDS, AIDS-related complex (ARC), adult T-cell leukemia (ATL) or Kawasaki disease.

**Revendications**
**Revendications pour les Etats contractants suivants : AT, BE, CH, DE, FR, GB, IT, LI, LU, NL, SE**

1. Utilisation d'une composition comprenant (a) un agent antiviral du type acide nucléique et (b) un glycoside acylé par un S-oxoacide, ce glycoside étant choisi parmi les glycosides de phénols, les glycosides de nitriles, les glycosides de coumarine, les glycosides de l'anthracène, les glycosides du terpène, les glycosides de l'amertume, les glycosides de la flavone, les glycosides de l'isoflavone, les glycosides du flavonol, les glycosides de la flavanone, les glycosides de la pélargonidine, les glycosides de cyanidine, les glycosides de la delphinidine, les glycosides de stéroïdes, les glycosides de triterpénïodes, les glycosides cardiotoniques, les glycosides de l'indoxyle, les glycosides de la gibberelline, les S-glycosides, les C-glycosides, et les glycosides mixtes C,O, ou d'un sel pharmaceutiquement acceptable de ces glycosides pour la fabrication d'un médicament pour le traitement d'une maladie causée par un rétrovirus.

2. Utilisation selon la revendication 1, dans laquelle cet agent antiviral est présent dans une quantité inférieure à sa dose antiviralement efficace par elle-même.

3. Utilisation selon la revendication 1, dans laquelle cet agent antiviral du type acide nucléique est choisi parmi la 3'-azido-3'-désoxythymidine (AZT), la 2',3'-didésoxythymidine, la 2',3'-didésoxyadénosine, la 2',3'-didésoxyguanosine, la 2',3'-didésoxyinosine, la 2',3'-didésoxycytidine, l'Ara A, l'Ara C, l'Ara T, l'iododésoxyuridine et l'Acyclovir.

4. Utilisation selon la revendication 1, dans laquelle au plains deux radicaux saccharides sont présents successivement dans le glycoside.

5. Utilisation selon la revendication 1, dans laquelle le glycoside est choisi parmi (i) un glycoside de triperpénoïdes, (ii) un glycoside de stéroïdes et (iii) un glycoside de terpène.

6. Utilisation selon la revendication 5, dans laquelle ce glycoside est choisi parmi (i) la glycyrrhizine, (ii) la digitonine et (iii) le stévioside.

7.  Utilisation selon la revendication 1, dans laquelle ce groupe S-oxoacide est un groupe sulfo (-SO$_3$H).

8.  Utilisation selon la revendication 1, dans laquelle le glycoside contient un groupe oxy (-O-) comme groupe de liaison entre l'aglycone et le radical du saccharide.

9.  Utilisation selon la revendication 1, dans laquelle le rétrovirus est un rétrovirus humain.

10. Utilisation selon la revendication 9, dans laquelle le rétrovirus humain est choisi parmi le HTLV-I, le HTLV-II, le HTLV-III, le LAV, l'ARV et le virus de la maladie de Kawasaki.

11. Utilisation selon la revendication 1, dans laquelle la maladie virale est le syndrome de l'adénopathic (LAS), l'adénopathic généralisée persistante (PGL), le SIDA, le complexe lié au SIDA (ARC), la leucémie des lymphocytes T de l'adulte (ATL), ou la maladie de Kawasaki.

12. Utilisation selon la revendication 1, dans laquelle cet agent antiviral du type acide nucléique (a) et ce glycoside (b) sont administrés simultanément et/ou successivement.

**Revendications pour les Etats contractants suivants : ES, GR**

1.  Procédé de préparation d'une composition pour le traitement d'une maladie causée par un rétrovirus, qui comprend le mélange (a) d'un agent anti-viral du type acide nucléique et (b) d'un glycoside acylé par un S-oxoacide, ce glycoside étant choisi parmi les glycosides de phénols, les glycosides de nitriles, les glycosides de coumarine, les glycosides de l'anthracène, les glycosides du terpène, les glycosides de l'amertume, les glycosides de la flavone, les glycosides de l'isoflavone, les glycosides du flavonol, les glycosides de la flavanone, les glycosides de la pélargonidine, les glycosides de cyanidine, les glycosides de la delphinidine, les glycosides de stéroïdes, les glycosides de triterpénoïdes, les glycosides cardiotoniques, les glycosides de l'indoxyle, les glycosides de la gibberelline, les S-glycosides, les C-glycosides, et les glycosides mixtes C,O ou d'un sel pharmaceutiquement acceptable de ces glycosides.

2.  Procédé selon la revendication 1, dans lequel cet agent antiviral est présent dans une quantité inférieure à sa dose antiviralement efficace par elle-même.

3.  Procédé selon la revendication 1, dans lequel set agent antiviral du type acide nucléique est choisi parmi la 3'-azido-3'-désoxythymidine (AZT), la 2',3'-didèsoxythymidine, la 2',3'-didésoxyadénosine, la 2',3'-didésoxyguanosine, la 2',3'-didésoxyinosine, la 2',3'-didésoxycytidine, l'Ara A, l'Ara C, l'Ara T, l'iododésoxyuridine et l'Acyclovir.

4.  Procédé selon la revendication 1, dans lequel au moins deux radicaux saccharides sont présents successivement dans le glycoside.

5.  Procédé selon la revendication 1, dans lequel le glycoside est choisi parmi (i) un glycoside de triperpénoïdes, (ii) un glycoside de stéroïdes et (iii) un glycoside de terpène.

6.  Procédé selon la revendication 5, dans lequel ce glycoside est choisi parmi (i) la glycyrrhizine (ii) la digitonine et (iii) le stévioside.

7.  Procédé selon la revendication 1, dans lequel ce groupe S-oxoacide est un groupe sulfo (-SO$_3$H).

8.  Procédé selon la revendication 1, dans lequel le glycoside contient un groupe oxy (-O-) comme groupe de liaison entre l'aglycone et le radical du saccharide.

9.  Procédé selon la revendication 1, dans lequel le rétrovirus est un rétrovirus humain.

10. Procédé selon la revendication 9, dans lequel le rétrovirus humain est choisi parmi le HTLV-I le HTLV-II, le HTLV-III, le LAV, l'ARV et le virus de la maladie de Kawasaki.

11. Procédé selon la revendication 1, dans lequel la maladie virale est le syndrome de l'adénopathie (LAS), l'adénopathie généralisée persistante (PGL), le SIDA, le complexe lié au SIDA (ARC), la leucémie des lymphocytes T de l'adulte (ATL), ou la maladie de Kawasaki.

**Patentansprüche**

**Patentansprüche für folgende Vertragsstaaten : AT, BE, CH, DE, FR, GB, IT, LI, LU, NL, SE**

1. Verwendung eines Mittels, das (a) ein antivirales Mittel des Nucleinsäure-Typs und (b) ein Glycosid, acyliert mit S-Oxosäure, enthält, wobei das Glycosid ausgewählt wird unter Phenolglycosiden, Nitrilglycosiden, Cumaringlycosiden, Anthracenglycosiden, Terpenglycosiden, Bitterkeitglycosiden, Flavonglycosiden, Isoflavonglycosiden, Flavonolglycosiden, Flavanoeglycosiden, Pelargonidinglycosiden, Cyanidinglycosiden, Delphinidinglycosiden, Steroidglycosiden, Triterpenoidglycosiden, cardiotonischen Glycosiden, Indoxylglycosiden, Gibberellinglycosiden, S-Glycosiden, C-Glycosiden und C,O-gemischten Glycosiden oder einem pharmazeutisch annehmbaren Salz solcher Glycoside, für die Herstellung eines Arzneimittels für die Behandlung einer Krankheit, die durch einen Retrovirus verusacht wird.

2. Verwendung nach Anspruch 1, wobei das antivirale Mittel in einer Menge vorhanden ist, die niedriger ist als die antiviral wirksame Menge selbst.

3. Verwendung nach Anspruch 1, wobei das antivirale Mittel des Nucleinsäure-Typs ausgewählt wird unter 3'-Azido-3'-desoxythymidin (AZT), 2',3'-Didesoxythymidin, 2',3'-Didesoxyadenosin, 2',3'-Didesoxyguanosin, 2',3'-Didesoxyinosin, 2',3'-Didesoxycytidin, Ara A, Ara C, Ara T, Ioddesoxyuridin und Acyclovir.

4. Verwendung nach Anspruch 1, wobei nicht weniger als zwei Saccharidreste in dem Glycosid aufeinanderfolgend vorhanden sind.

5. Verwendung nach Anspruch 1, wobei das Glycosid ausgewählt wird unter (i) Triterpenoidglycosid, (ii) Steroidglycosid und (iii) Terpenglycosid.

6. Verwendung nach Anspruch 5, wobei das Glycosid ausgewählt wird unter (i) Glycyrrhizin, (ii) Digitonin und (iii) Steviosid.

7. Verwendung nach Anspruch 1, wobei die S-Oxosäuregruppe die Sulfogruppe (-SO$_3$H) ist.

8. Verwendung nach Anspruch 1, wobei das Glycosid eine Oxogruppe (-O-) als Verbindungsgruppe zwischen dem Aglycon- und dem Saccharidrest enthält.

9. Verwendung nach Anspruch 1, wobei das Retrovirus humanes Retrovirus ist.

10. Verwendung nach Anspruch 9, wobei das humane Retrovirus ausgewählt wird unter HTLV-I, HTLV-II, HTLV-III, LAV, ARV und dem Virus der Kawasaki-Krankheit.

11. Verwendung nach Anspruch 1, wobei die virale Krankheit das Lymphadenopathie-Syndrom (LAS), persistente generalisierte Lymphadenopathie (PGL), AIDS, AIDS-verwandter Komplex (ARC), T-Zellen-Leukämie (ATL) bei Erwachsenen oder Kawasaki-Krankheit ist.

12. Verwendung nach Anspruch 1, wobei das antivirale Mittel des Nucleinsäure-Typs (a) und das Glycosid (b) gleichzeitig und/oder aufeinanderfolgend verabreicht werden.

**Patentansprüche für folgende Vertragsstaaten : ES, GR**

1. Verfahren zur Herstellung eines Mittels für die Behandlung einer Krankheit, die durch einen Retrovirus verursacht wird, dadurch **gekennzeichnet,** daß (a) ein antivirales Mittel des Nucleinsäure-Typs und (b) ein Glycosid, acyliert mit S-Oxosäure, wobei das Glycosid ausgewählt wird unter Phenolglycosiden, Nitrilglycosiden, Cumaringlycosiden, Anthracenglycosiden, Terpenglycosiden, Bitterkeitglycosiden, Flavonglycosiden, Isoflavonglycosiden, Flavonolglycosiden, Flavanoeglycosiden, Pelargonidinglycosiden, Cyanidinglycosiden, Delphinidinglycosiden, Steroidglycosiden, Triterpenoidglycosiden, cardiotonischen Glycosiden, Indoxylglycosiden, Gibberellinglycosiden, S-Glycosiden, C-Glycosiden und C,O-gemischten Glycosiden oder einem pharmazeutisch annehmbaren Salz solcher Glycoside, vermischt werden.

**2.** Verfahren nach Anspruch 1, dadurch **gekennzeichnet,** daß das antivirale Mittel in einer Menge vorhanden ist, die niedriger ist als die antiviral wirksame Menge selbst.

**3.** Verfahren nach Anspruch 1, dadurch **gekennzeichnet,** daß das antivirale Mittel des Nucleinsäure-Typs ausgewählt wird unter 3'-Azido-3'-desoxythymidin (AZT), 2',3'-Didesoxythymidin, 2',3'-Didesoxyadenosin, 2',3'-Didesoxyguanosin, 2',3'-Didesoxyinosin, 2',3'-Didesoxycytidin, Ara A, Ara C, Ara T, Ioddesoxyuridin und Acyclovir.

**4.** Verfahren nach Anspruch 1, dadurch **gekennzeichnet,** daß nicht weniger als zwei Saccharidreste in dem Glycosid aufeinanderfolgend vorhanden sind.

**5.** Verfahren nach Anspruch 1, dadurch **gekennzeichnet,** daß das Glycosid ausgewählt wird unter (i) Triterpenoidglycosid, (ii) Steroidglycosid und (iii) Terpenglycosid.

**6.** Verfahren nach Anspruch 5, dadurch **gekennzeichnet,** daß das Glycosid ausgewählt wird unter (i) Glycyrrhizin, (ii) Digitonin und (iii) Steviosid.

**7.** Verfahren nach Anspruch 1, dadurch **gekennzeichnet,** daß die S-Oxosäuregruppe die Sulfogruppe (- $SO_3H$) ist.

**8.** Verfahren nach Anspruch 1, dadurch **gekennzeichnet,** daß das Glycosid eine Oxogruppe (-O-) als Verbindungsgruppe zwischen dem Aglycon- und dem Saccharidrest enthält.

**9.** Verfahren nach Anspruch 1, dadurch **gekennzeichnet,** daß das Retrovirus humanes Retrovirus ist.

**10.** Verfahren nach Anspruch 9, dadurch **gekennzeichnet,** daß das humane Retrovirus ausgewählt wird unter HTLV-I, HTLV-II, HTLV-III, LAV, ARV und dem Virus der Kawasaki-Krankheit.

**11.** Verfahren nach Anspruch 1, dadurch **gekennzeichnet,** daß die virale Krankheit das Lymphadenopathie-Syndrom (LAS), persistente generalisierte Lymphadenopathie (PGL), AIDS, AIDS- verwandter Komplex (ARC), T-Zellen-Leukämie (ATL) bei Erwachsenen oder Kawasaki-Krankheit ist.